Europäisches Patentamt

European Patent Office

Office européen des brevets

(11) Publication number: **0 279 244**
**A2**

## EUROPEAN PATENT APPLICATION

(21) Application number: **88101227.2**

(22) Date of filing: **28.01.88**

(51) Int. Cl.4: **A61K 7/06**

(30) Priority: **06.02.87 IT 1927787**

(43) Date of publication of application:
**24.08.88 Bulletin 88/34**

(84) Designated Contracting States:
**AT BE CH DE ES FR GB GR IT LI LU NL SE**

(71) Applicant: **TONFER INC.**
**22 Crestmont Road**
**Montclair New Jersey 07042(US)**

(72) Inventor: **Ferro, Antonio**
**via Maraini 7**
**CH-6900 Lugano(CH)**

(74) Representative: **Dragotti, Gianfranco et al**
**SAIC BREVETTI s.r.l. Viale Bianca Maria, 15**
**I-20122 Milano(IT)**

(54) **A composition for the treatment and stimulation of the hair growth.**

(57) The topical application of a composition comprising, as the active ingredient, a compound selected among heparin and/or vitamin K, and/or clofibrate stimulates the hair follicle, with specific action of inhibition of the hair fall, with anti-dandruff and anti-seborrhea action, and with stimulation of the hair regrowth.

EP 0 279 244 A2

## "The composition for the treatment and stimulation of the hair growth"

A present invention relates to a composition for the stimulation of the hair follicle and thus for the stimulation of the hair growth and regrowth.

The importance of this problem does not need particular comments and has been since ever the objective of the research in the cosmetic and paramedical field.

To this situation a further consideration must be added: some drugs, which became of wide use in recent years, for instance for the anti-tumoral therapy, cause the very quick and irreparable fall of the hair, whereby the problem, if possible, became more and more serious.

On the other side the present heavy atmospherical pollution contributes to such a worsening of the situation.

It has been now found that some active principles, per se known, if topically applied, have the property of acting on the hair follicle promoting the hair regrowth and showing a controlling action on the hair fall and an anti-dandruff and anti-seborrhea activity.

It has been particularly found that vitamin $K_1$, heparin and clofibrate, both alone and mainly in combination with each other show the aforesaid activity, whereby they constitute the active ingredients of the compositions forming the object of the present invention.

For the better understanding of the invention there are firstly reported the data relating to the experiments carried out in the animal and in the human being.

A) Test on the animal.

These experiments have been carried out on Burgundi reddish rabbits, the back of which was shaved, carring out the administration in double manner, or in comparison with a placebo.

1) Test of hair regrowth by means of intradermal injection.

The results of these tests are reported in following table I, in which:
- no regrowth;
+ -just remarkable regrowth;
+ remarkable regrowth;
+ + abundant regrowth;
+ + + very abundant and intense regrowth.

## TABLE 1

### HAIR REGROWTH

| | After 7 days | after 15 days | after 30 days |
|---|---|---|---|
| Heparin 0.1% | +− | + | ++ |
| Heparin 0.01% | − | +− | + |
| Vitamin $K_1$ 0.1% | − | + | ++ |
| Vitamin $K_2$ 0.1% | − | +− | + |
| Vitamin $K_3$ 0.1% (sodium menadione bisulfite) | irritating | irritating | irritating |
| Vitamin $K_5$ 0.1% | − | +− | ++ |
| Heparin 0.1 + Vitamin $K_1$ 0.1% | + | ++ | +++ |

From the preceding table it can be appreciated that, although in a different degree, all the vitamins K stimulate the regrowth of the hair with delayed times with respect to heparin, but vitamin $K_3$ is irritating. The combination of heparin with vitamin K, has shown a synergy both improving the regrowth time and as regards thickness and the lenght of regrown hair.

It has not been possible to test of the activity of the clofibrate by injective route.

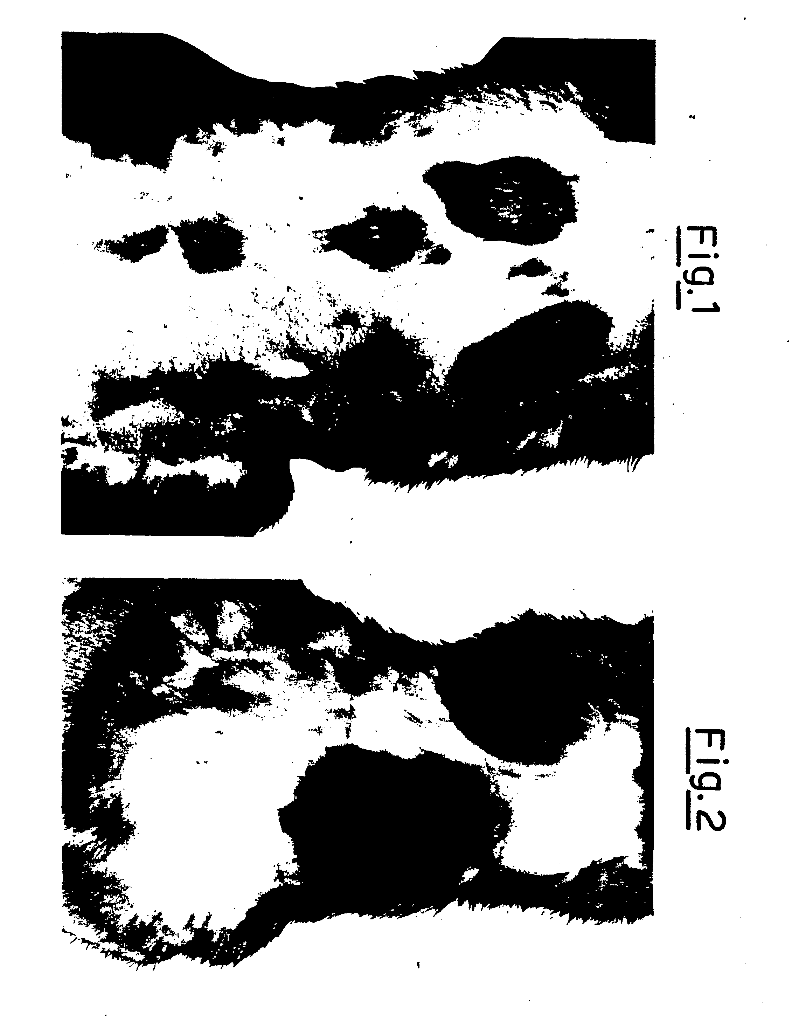

In the enclosed fig. 1 the photographic picture is shown of the back of a rabbit 30 days after the starting of the treatment.

In this figure it can be seen that the treatment has been carried out in double manner and more precisely:

| Areas | injected composition |
|---|---|
| 1 = | heparin 0.1% + vitamin $K_1$ 0.1% |
| 2 = | vitamin $K_1$ 0.1% |
| 3 = | heparin 0.1% |
| 4 = | heparin 0.01% |

(2) Test of hair regrowth by means of topical applications.

The results of these tests are reported in the following table 2, in which it can be seen that clofibrate and vitamin K, intensely stimulate the hair regrowth whereas the action of heparin seems slighter under the same experimental conditions.

In turn vitamin $K_3$ ,although stimulates the regrowth, is irritating also in topical applications.

In that case it can be noted that the combinations of heparin with clofibrate and/or vitamin K, as well as that of clofibrate and vitamin K, stimulate the hair growth in an improved manner with undeniable indicia of synergic activity.

## TABLE 2

### HAIR REGROWTH

|  | after 15 days | after 30 days | after 60 days |
|---|---|---|---|
| Heparin 0.1% | − | − | +− |
| Vitamin $K_1$ 1% | − | +− | + |
| Vitamin $K_3$ 1% (sodium menadione bisulfite) 1% | − | ++ | ++ |
| Clofibrate 2% | − | ++ | ++ |
| Fenfibrate 2% | − | − | +− |
| Benzafibrate 2% | − | − | − |
| Etofibrate 2% | − | − | +− |
| Clofibride 2% | − | +− | +− |
| Heparin 0.1% + Clofibrate 2% | − | + | ++ |
| Heparin 0.01% + Vitamin $K_1$ 1% | − | +− | +++ |
| Vitamin $K_1$ 1% + Clofibrate 2% | − | ++ | ++ |
| Heparin 0.1% + Vitamin $K_1$ 1% | − | +− | +++ |
| Vitamin $K_1$ % + Clofibrate 2% | − | ++ | ++ |
| Heparin 0.1% + Clofibrate 2%+ Vitamin $K_1$ 1% | − | ++ | +++ |

In figure 2 the photographic picture is shown of the back of a rabbit on which the experiment has been carried out.

In that case each active component or combination of active components is compared with placebo.

B) Test in the human being.

On the base of the tests carried out in the rabbit the compositions of the invention have been tested for the alopecia. The tests in the human being have been carried out exclusively by topical route through daily application on the head cutis of the tested preparation for two months and carrying out the evaluation after 30,60,90 and 120 days.

The following parameters have been taken into account.
- hair fall;
- dandruff , if present;
- sebum, if in excess;
- hair regrowth, examination after 120 days.

There have been used lotions containing 15% of ethyl alcohol and 0.15% of nipagin and the following active ingredients:

A) Clofibrate 2% (10 patients treated)
B) Heparin 0.1% + Vitamin K, 0.1 (10 patients treated)
C) Heparin 0.1% + Vitamin K, 0.1% + clofibrate 2% (10 patients treated).


Results:

Lotion A (Clofibrate 2%)
- good activity against hair fall;
- good activity against dandruff and seborrhea
- no regrowth in the observation period
Lotion B (heparin 0.1% + Vitamin K, 0.1%).
- Optimum action against hair fall
- good anti-dandruff and anti-seborrhea activity;
- evident regrowth in four patients.
Lotion C (heparin 0.1% + Vitamin K, 0.1% + Clofibrate 2%).
- Optimum activity against hair fall;
- good anti-dandruff activity;
- good sebum controlling activity;
- regrowth of hair in 5 patients.

From the results of the preceeding tests it is evident that the above indicated compounds both alone and above all in combination are able to stimulate the hair growth in the animal and of efficaciously and positively acting in the human alopecia by influencing several parameters such as the hair fall, dandruff, seborrhea and hair regrowth.

Such an activity is even more surprising since there are involved substances which were per se already known and used in the human therapy but such a property thereof had not been detected or even suggested.

By more specifically considering these substances:

a) heparin, known since 1922, is defined as a mucopolysaccharide mainly consisting of residues of D-glucosamine and of D-glucuronic acid. The specific use of this substance is as anti-coagulating drug employed in supporting the post-surgical therapy, to be administered by systemic route, mainly by injecting route.

A specific property of heparin referred to in the literature is that the same, if administered at doses at which it shows an anti-coagulating effect causes as side effect the hair fall.

(b) Vitamin K, (2-methyl-3-phytyl-1,4-nathtoquinone) is also known since 1929 and the activity thereof has been described as anti-hemorrhagical, namely to promote the coagulation of blood.

The specific therapeutical indication is that of the treatment in prophlaxis of hypoprotrombinemia.

(c) Clofibrate, ethylester of (cloro-4'-thenoxy)-2-isobutyric acid is known as hypolipemizing agent for systemic use.

From the above short notes it is evident that the above described effects, of the invention as confirmed from the experimental tests, are totally novel and surprising.

Much more surprising is the fact that these substances, so different as to chemical structure and therapeutical activity, show a relevant synergy as regards the stimulation of the hair follicle and as regards the other above mentioned properties.

From the experimental tests it has been also shown that the dosages of use of the above substances in the compositions according to the present invention are comprised in the following ranges:
- clofibrate      0.5 - 5%
- vitamin K,      0.010-1%
- heparin         0.005-01%

5

Some examples of formulations according to the invention are now given, it being meant that they have exemplifying but non limiting purposes. In the formulations the percentages are expressed by weight referred to the total weight of the composition.

1) Clofibrate 2
95° Ethyl alcohol 30
$H_2O$ enough

2) Vitamin K, 0.1
Propylene glycol 5
Nipagin 0.1
$H_2O$ enough

3) Clofibrate 1.5
95° Ethyl alcohol 20
Heparin 0.03
$H_2O$ enough

4) Vitamin K, 0.050
Propylene glycole 2.5
Heparin 0.050
$H_2O$ enough

5) Vitamin K, 0.05
Clofibrate 2
95° Ethyl alcohol 20
$H_2O$ enough

6) Vitamin K, 0.03
Clofibrate 2
Heparine 0.010
$H_2O$ enough

it is to be noted that the formulations may contain other usual components, such as for example perfumes and essences or other agents improving physical or use properties.

## Claims

1. A composition having stimulating activity of the hair follicle for topical use, characterized by containing, as the active ingredient, at least a compound selected amoung heparin, vitamin K, and clofibrate together with at least an excipient or vehicle.

2. A composition according to claim 1 characterized in that the excipient or vehicles is selected among water, alcohols, glycols or their mixtures.

3. A composition according to claim 2, characterized in that said alcohol is ethanol and said glycol is propylene glycol.

4. A composition according to claim 1, characterized by containing 0.005-0.1% by weight of heparin.

5. A composition according to claim 1, characterized by containing 0.010-1% by weight of vitamin K,.

6. A composition according to claim 1, characterized by containing 0.5-5% by weight of clofibrate.

7. A composition according to claim 1, characterized by containing, as the active ingredient, heparin and another component selected among vitamin K, and clofibrate.

8. A composition according to claim 7, characterized by the fact said heparin and said other component are present in the amounts respectively indicated in the claims 4,5 and 6.

9. A composition according to claim 1, characterized by containing in combination as active ingredients: heparin, vitamin K, and clofibrate.

10. Use of a compound selected among heparin, vitamin K, clofibrate, and their mixtures, for the preparation of a composition for topical application for the stimulation of hair follicle.

11. Use according to claim 10, wherein said compound is present in the amounts of claims 4, 5 and 6.

Fig.1

Fig.2